(19) Europäisches Patentamt
European Patent Office
Office européen des brevets

(11) Veröffentlichungsnummer: **0 096 354**
**B1**

(12) # EUROPÄISCHE PATENTSCHRIFT

(45) Veröffentlichungstag der Patentschrift: **08.10.86**

(21) Anmeldenummer: **83105407.7**

(22) Anmeldetag: **01.06.83**

(51) Int. Cl.[4]: **C 07 D 401/12**, C 07 D 213/64, C 07 F 9/58, A 01 N 43/64, A 01 N 43/40

(54) **Substituierte Pyridyl-phenyl-ether.**

(30) Priorität: **04.06.82 DE 3221214**
**04.01.83 DE 3300141**

(43) Veröffentlichungstag der Anmeldung: **21.12.83 Patentblatt 83/51**

(45) Bekanntmachung des Hinweises auf die Patenterteilung: **08.10.86 Patentblatt 86/41**

(84) Benannte Vertragsstaaten: **AT BE CH DE FR GB IT LI NL**

(56) Entgegenhaltungen:
**EP - A - 0 003 313**
**EP - A - 0 008 624**
**EP - A - 0 032 561**
**EP - A - 0 044 163**
**AU - A - 496 882**
**DE - A - 2 732 846**
**DE - B - 2 714 662**

**Chemie der Pflanzenschutz-und Schädlingsbekämpfungsmittel, Band 8, Springer-Verlag Berlin, 1982, Seiten 2-9**

(73) Patentinhaber: **BAYER AG, Konzernverwaltung RP Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Klauke, Erich, Dr., Eichendorffweg 8, D-5068 Odenthal (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 096 354 B1

## Beschreibung

Die Erfindung betrifft neue, substituierte Pyridyl-phenylether, mehrere Verfahren zu deren Herstellung sowie ihre Verwendung als Herbizide.

Es ist bereits bekannt geworden, dass zahlreiche Phenoxypropionsäure-Derivate herbizide Eigenschaften besitzen (vgl. DE-OS 2 223 894 und DE-OS 2 812 571, DE-A 2 732 846, EP-A-8624 und R. Wegler, Chemie der Pflanzenschutz- und Schädlingsbekämpfungsmittel, Band 8, Springer Verlag, Berlin 1982, S. 2-9).

So können zum Beispiel der 2-[4-(2,4-Dichlorphenoxy)-phenoxy]-propionsäure-methylester, der 2-[4-(5-Trifluormethyl-pyridyl-2-oxy)-phenoxy]-propionsäure-ethylester und der [4-(5-Trifluormethyl-pyridyl-2-oxy)-phenoxy]-essigsäure-ethylester zur Unkrautbekämpfung eingesetzt werden. Die Wirkung dieser Stoffe ist jedoch insbesondere bei einigen Gräsern und beim Einsatz niedriger Aufwandmengen nicht immer ausreichend.

Es wurden nun neue substituierte Pyridyl-phenyl-ether der Formel

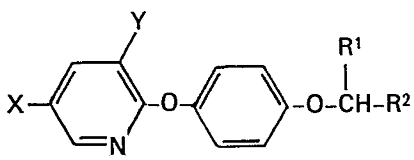
(I)

in welcher
X für Trifluormethyl oder Chlor steht,
Y für Wasserstoff oder Chlor steht,
$R^1$ für Wasserstoff oder Methyl steht und
$R^2$ für den Rest der Formel

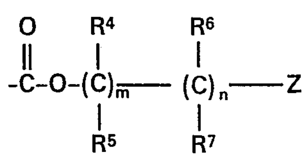

steht, in welcher
$R^4$, $R^5$, $R^6$ und $R^7$ unabhängig voneinander für Wasserstoff oder Alkyl mit 1 bis 4 Kohlenstoffatomen stehen,
15 m für 1 oder 2 steht,
n für 0 oder 1 steht und
Z für Trimethylsilyl steht,
gefunden.

Diejenigen substituierten Pyridyl-phenylether der Formel (I), in denen $R^1$ für Methyl steht, enthalten ein asymmetrisches Kohlenstoffatom in der Seitenkette und können deshalb in zwei enantiomeren Formen vorliegen. Die Erfindung betrifft sowohl die jeweiligen Racemate als auch die R- und S-Enantiomeren.

Unter R-Enantiomeren (S-Enantiomeren) sind hierbei jeweils diejenigen optisch aktiven Verbindungen zu verstehen, welche am asymmetrischen Kohlenstoffatom der Propionsäure-Einheit die R-Konfiguration (S-Konfiguration) aufweisen.

Weiterhin wurde gefunden, dass man

a) substituierte Pyridyl-phenyl-ether der Formel (I) erhält, wenn man 4-(Pyridyl-3-oxy)-phenole der Formel

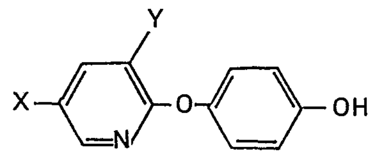
(II)

in welcher
X und Y die oben angegebene Bedeutung haben,
mit Alkancarbonsäure-Derivaten der Formel

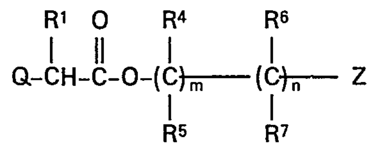
(III)

in welcher
$R^1$, $R^4$, $R^5$, $R^6$, $R^7$, Z, m und n die oben angegebene Bedeutung haben und
Q für Chlor, Brom, Mesylat oder Tosylat steht,
gegebenenfalls in Gegenwart eines Säurebindemittels und gegebenenfalls in Gegenwart eines Verdünnungsmittels umsetzt, oder

b) die substituierten Pyridyl-phenyl-ether der Formel (I) erhält, wenn man Phenoxyalkancarbonsäure-Derivate der Formel

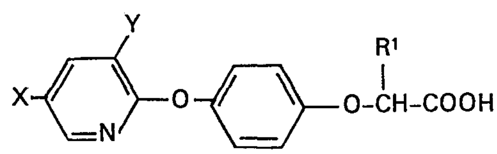
(IV)

in welcher
X, Y und $R^1$ die oben angegebene Bedeutung haben,
mit Silylchloriden der Formel

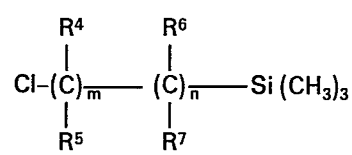
(V)

in welcher
$R^4$, $R^5$, $R^6$, $R^7$, m und n die oben angegebene Bedeutung haben,
gegebenenfalls in Gegenwart eines Säurebindemittels sowie gegebenenfalls in Gegenwart eines Verdünnungsmittels umsetzt.

Schliesslich wurde gefunden, dass sich die neuen substituierten Pyridyl-phenyl-ether der Formel (I) durch eine hervorragende herbizide Wirksamkeit auszeichnen.

Überraschenderweise besitzen die erfindungsgemässen substituierten Pyridyl-phenyl-ether der Formel (I) bessere herbizide Eigenschaften als der aus dem Stand der Technik bekannte 2-[4-(2,4-Dichlorphenoxy)-phenoxy]-propionsäure-methylester, welcher ein hochwirksamer Wirkstoff gleicher Wirkungsart ist. Vor allem lassen sich mit Hilfe der erfindungsgemässen Wirkstoffe einige Ungräser, die von dem 2-[4-(2,4-Dichlorphenoxy)-phenoxy]-propionsäuremethylester nicht erfasst werden, wirksam bekämpfen. Darüber hinaus übertreffen die erfindungsgemässen substituierten Pyridyl-phenyl-ether hinsichtlich der selektiven herbiziden Potenz auch den 2-[4-(5-Trifluormethyl-pyridyl-2-oxy)-phenoxy]-propionsäure-ethylester und den [4-(5-Trifluormethylpyridyl-2-oxy)- phenoxy]-essigsäure-ethylester, welches konstitutionell ähnliche Wirkstoffe analoger Wirkungsrichtung sind.

Die erfindungsgemässen substituierten Pyridyl-phenylether sind durch die Formel (I) eindeutig definiert. In dieser Formel steht X vorzugsweise für Trifluormethyl oder Chlor, und Y steht vorzugsweise für Wasserstoff oder Chlor, wobei Y insbesondere dann für Wasserstoff steht, wenn X für Trifluormethyl steht, und Y insbesondere dann für Chlor steht, wenn X auch für Chlor steht. $R^1$ steht vorzugsweise für Wasserstoff oder Methyl, und $R^2$ steht für den Rest der Formel

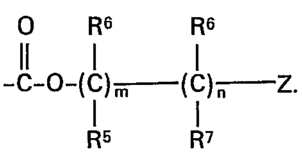

In diesem Rest stehen $R^4$, $R^5$, $R^6$ und $R^7$ unabhängig voneinander vorzugsweise für Wasserstoff oder Methyl. Der Index m steht vorzugsweise für 1 oder 2 und der Index n steht vorzugsweise für 0 oder 1. Der Substituent Z steht für Trimethylsilyl.

Eine weitere Gruppe von besonders bevorzugten erfindungsgemässen Verbindungen sind diejenigen Stoffe der Formel (I), in denen X für Trifluormethyl und Y für Wasserstoff steht, oder in denen X und Y gleichzeitig für Chlor stehen, $R^1$ für Wasserstoff oder Methyl steht und $R^2$ für die Gruppierung der Formel

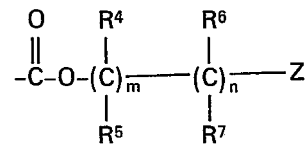

steht, wobei $R^4$, $R^5$, $R^6$, $R^7$, m und n diejenigen Bedeutungen haben, die oben bereits vorzugsweise für diese Reste und Indices genannt wurden, und wobei Z für Trimethylsilyl steht.

Besonders bevorzugt sind auch die R-Enantiomeren derjenigen substituierten Pyridyl-phenylether der Formel (I), in denen $R^1$ für Methyl steht, also die erfindungsgemässen Verbindungen der Formel

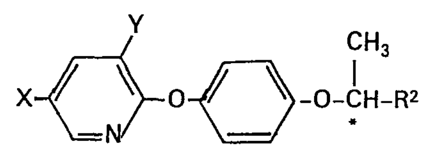 (Ib)

in welcher

X, Y und $R^2$ die oben als bevorzugt genannte Bedeutung haben.

In dieser Formel ist das asymmetrische Kohlenstoffatom (Asymmetriezentrum), das die R-Konfiguration aufweist, durch ein (*) gekennzeichnet.

Als Beispiele für substituierte Pyridyl-phenylether der Formel (I) seien die in der folgenden Tabelle formelmässig aufgeführten Verbindungen genannt.

Tabelle 1

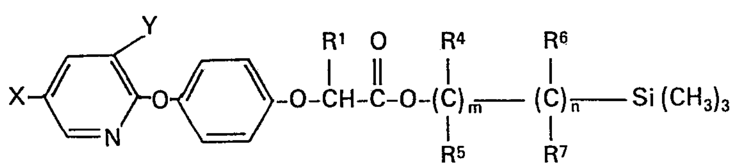

| X | Y | $R^1$ | $R^4$ | $R^5$ | $R^6$ | $R^7$ | m | n |
|---|---|---|---|---|---|---|---|---|
| $CF_3$ | H | H | H | H | H | H | 1 | 1 |
| $CF_3$ | H | H | H | H | – | – | 1 | 0 |
| $CF_3$ | H | $CH_3$ | H | H | H | H | 1 | 1 |
| $CF_3$ | H | $CH_3$ | H | H | – | – | 1 | 0 |
| Cl | Cl | H | H | H | H | H | 1 | 1 |
| Cl | Cl | H | H | H | – | – | 1 | 0 |
| Cl | Cl | $CH_3$ | H | H | H | H | 1 | 1 |
| Cl | Cl | $CH_3$ | H | H | – | – | 1 | 0 |

Verwendet man 4-(5-Trifluormethyl-pyridyl-2-oxy)-phenol und 2-Tosyloxy-propionsäure-2-trimethylsilylester als Ausgangsstoffe, so kann der Verlauf des erfindungsgemässen Verfahrens (a) durch das folgende Formelschema wiedergegeben werden.

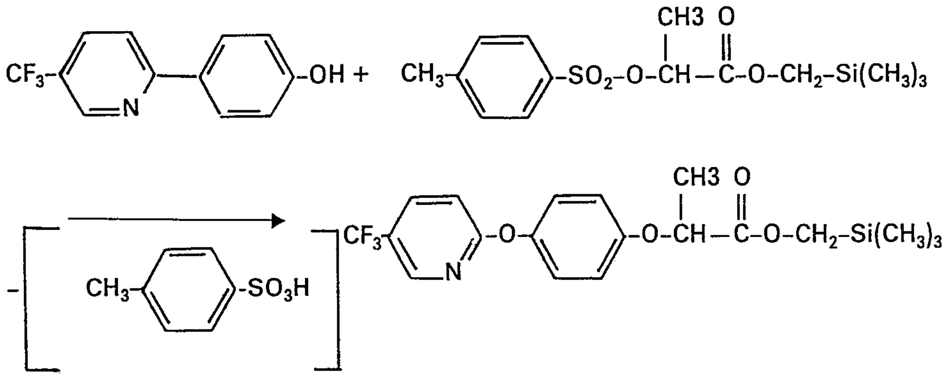

Verwendet man 2-[4-(5-Trifluormethyl-pyridyl-2-oxy)-phenoxy]-propionsäure und Chlormethyltrimethylsilan als Ausgangsstoffe, so kann der Verlauf des erfindungsgemässen Verfahrens (b) durch das folgende Formelschema widergegeben werden:

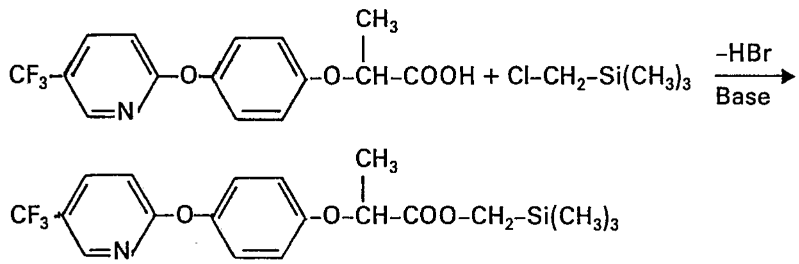


Die bei dem erfindungsgemässen Verfahren (a) als Ausgangsmaterialien benötigten 4-(Pyridyl-2-oxy)-phenole sind durch die Formel (II) definiert. In dieser Formel haben X und Y diejenigen Bedeutungen, die bereits im Zusammenhang mit den erfindungsgemässen Stoffen der Formel (I) vorzugsweise X und Y genannt wurden.

Die 4-(Pyridyl-2-oxy)-phenole der Formel (II) sind bereits bekannt (vgl. DE-OS 2 812 571, DE-OS 2 758 002 und US-PS 4 046 553).

Die bei dem erfindungsgemässen Verfahren (a) als Ausgangsstoffe benötigten Alkancarbonsäure-Derivate sind durch die Formel (III) definiert. In dieser Formel haben $R^1$, $R^4$, $R^5$, $R^6$, $R^7$, Z, m und n vorzugsweise diejenigen Bedeutungen, die bereits im Zusammenhang mit der Beschreibung der erfindungsgemässen Stoffe der Formel (I) vorzugsweise für diese Reste und Indices genannt wurden.

Die Alkancarbonsäure-Derivate der Formel (III) sind bisher noch nicht bekannt. Sie lassen sich herstellen, indem man

c) Verbindungen der Formel

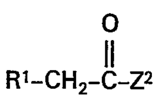

(VI)

in welcher
$R^1$ die oben angegebene Bedeutung hat und
$Z^2$ für Hydroxy oder Chlor steht,
mit Verbindung der Formel

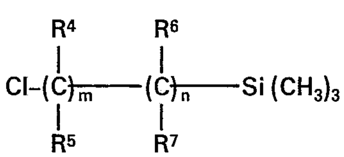

(V)

in welcher
$R^4$, $R^5$, $R^6$, $R^7$, m und n die oben genannte Bedeutung haben,
gegebenenfalls in Gegenwart eines Säurebindemittels sowie gegebenenfalls in Gegenwart eines Verdünnungsmittels umsetzt und die dabei entstehenden Stoffe der Formel

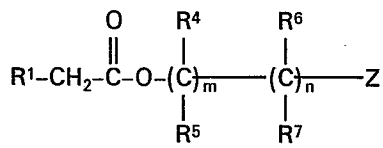

(VII)

in welcher
$R^1$, $R^4$, $R^5$, $R^6$, $R^7$, Z, m und n die oben angegebene Bedeutung haben,
nach üblichen Methoden in die Alkancarbonsäure-Derivate der Formel (III) überführt, oder
Verbindungen der Formel

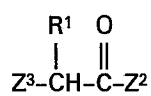

(VIII)

in welcher
$R^1$ und $Z^2$ die oben angegebene Bedeutung haben und
$Z^3$ für Mesylat oder Tosylat steht,
mit Verbindungen der Formel

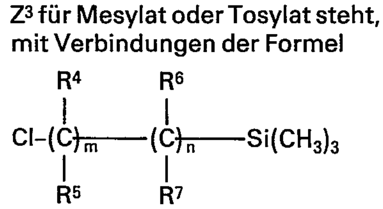

(V)

oder

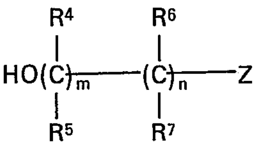 (IX)

in welchen
$R^4$, $R^5$, $R^6$, $R^7$, Z, m und n die oben angegebene Bedeutung haben,
gegebenenfalls in Gegenwart eines Säurebindemittels sowie gegebenenfalls in Gegenwart eines Verdünnungsmittels umsetzt.

Die Reaktionsbedingungen bei den oben erwähnten Verfahren (c) und (d) entsprechen denjenigen des erfindungsgemässen Verfahrens (a) (vgl. unten).

Zur Herstellung von optisch aktiven Verbindungen der Formel (I), in denen $R^1$ für Methyl steht, werden bei der Durchführung des erfindungsgemässen Verfahrens (a) die R- bzw. S-Enantiomeren der betreffenden Alkancarbonsäure-Derivate der Formel (III) benötigt. Dabei werden zur Synthese der R-Enantiomeren der Formel (I) die S-Enantiomeren der Alkancarbonsäure-Derivate der Formel (III) eingesetzt, und zur Synthese der S-Enantiomeren der Verbindungen der Formel (I) werden die R-Enantiomeren der Alkancarbonsäure-Derivate der Formel (III) eingesetzt. Das Ausgangsprodukt der Formel (III) hat am Asymmetriezentrum jeweils die entgegengesetzte Konfiguration wie das Endprodukt, da im Verlauf der Umsetzung eine Walden'sche Umkehr am Asymmetriezentrum erfolgt.

Die optisch aktiven Alkancarbonsäure-Derivate der Formel (III) lassen sich nach dem oben erwähnten Verfahren (d) herstellen, indem man die jeweiligen optisch aktiven Verbindungen der Formel (VIII) einsetzt.

Die bei dem erfindungsgemässen Verfahren (b) als Ausgangsstoffe benötigten Phenoxyalkancarbonsäure-Derivate sind durch die Formel (IV) definiert. In dieser Formel steht $R^1$ für Wasserstoff oder Methyl. X steht für Trifluormethyl oder Chlor, und Y steht für Wasserstoff oder Chlor.

Die Verbindungen der Formel (IV) sind bekannt (vgl. DE-OS 2 812 571 und US-PS 4 046 553).

Die bei dem erfindungsgemässen Verfahren (b) weiterhin als Ausgangsstoffe benötigten Silylchloride und Silylalkohole sind durch die Formel (V) und (IX) definiert. In dieser Formel haben $R^4$, $R^5$, $R^6$, $R^7$, m und n vorzugsweise diejenigen Bedeutungen, die bereits im Zusammenhang mit der Beschreibung der erfindungsgemässen Stoffe der Formel (I) vorzugsweise für diese Reste und Indices genannt wurden.

Als Beispiele für Verbindungen der Formel (V) seien die in der folgenden Tabelle 2 formelmässig aufgeführten Stoffe genannt:

Tabelle 2

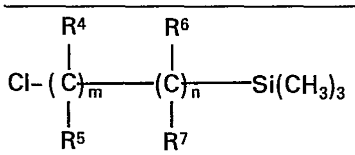 (V)

| $R^4$ | $R^5$ | $R^6$ | $R^7$ | m | n |
|---|---|---|---|---|---|
| H | H | – | – | 1 | 0 |
| H | H | H | H | 1 | 1 |
| H | H | $CH_3$ | $CH_3$ | 1 | 1 |
| H | H | H | H | 2 | 1 |

Die Silylchloride der Formel (V) und die Silylalkohole der Formel (IX) sind bekannt oder lassen sich in einfacher Weise nach bekannten Verfahren herstellen.

Zur Herstellung von R- und S-Enantiomeren der substituierten Pyridyl-phenyl-ether der Formel (I), in denen $R^1$ für Methyl steht, werden bei der Durchführung des erfindungsgemässen Verfahrens (b) optisch aktive R- oder S-Phenoxypropionsäure-Derivate der Formel (IV) als Ausgangsstoffe benötigt. Auch diese optisch aktiven Phenoxy-propionsäure-Derivate sind bekannt (vgl. DE-OS 2 758 002).

Man erhält die R- bzw. S-Enantiomeren der Phenoxy-propionsäure-Derivate der Formel

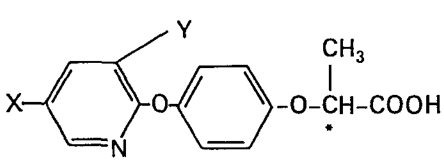 (IVa)

in welcher
X und Y die oben angegebene Bedeutung haben,
indem man 4-(Pyridyl-2-oxy)-phenole der Formel

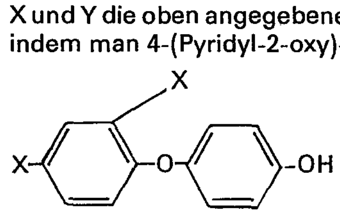 (II)

in welcher
X und Y die oben angegebene Bedeutung haben,
mit den S-Enantiomeren bzw. R-Enantiomeren von Propionsäure-Derivaten der Formel

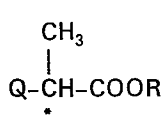 (X)

in welcher
Q die oben angegebene Bedeutung hat und
R für Methyl oder Ethyl steht,

gegebenenfalls in Gegenwart eines Säureakzeptors, wie z. B. Kaliumcarbonat, und gegebenenfalls in Gegenwart eines Verdünnungsmittels, wie zum Beispiel Acetonitril, bei Temperaturen zwischen 0°C und 120°C umsetzt und die dabei entstehenden Ester der Formel

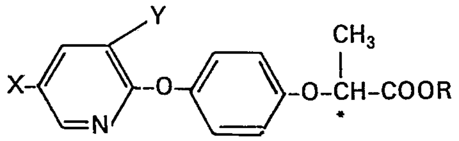

(XI)

in welcher

R, X und Y die oben angegebene Bedeutung haben,

mit starken Basen, wie zum Beispiel Natriumhydroxid, in Gegenwart eines Verdünnungsmittels, wie zum Beispiel Methanol, Ethanol, Benzol, Toluol, Xylol, gegebenenfalls im Gemisch mit Wasser bei Temperaturen zwischen 20°C und 140°C verseift und anschliessend mit einer Säure, wie zum Beispiel Salzsäure, ansäuert.

In der ersten Stufe dieser Umsetzung findet am asymmetrischen Kohlenstoffatom der Propionsäure-Einheit eine Walden'sche Umkehr statt.

Dieses hat zur Folge, dass durch Umsetzung von 4-(Pyridyl-2-oxy)-phenolen der Formel (II) mit den S-Enantiomeren der Propionsäure-Derivate der Formel (X) die R-Enantiomeren der Phenoxy-propionsäure-Derivate der Formel (IVa) entstehen. Andererseits bilden sich durch Umsetzung von 4-(Pyridyl-2-oxy)-phenolen der Formel (II) mit den R-Enantiomeren der Propionsäure-Derivate der Formel (X) die S-Enantiomeren der Phenoxypropionsäure-Derivate der Formel (IVa).

Die erfindungsgemässen Verfahren (a) und (b) zur Herstellung der neuen substituierten Pyridyl-phenyl-ether der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Tuluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methylisobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säurebindemittel können bei den erfindungsgemässen Verfahren (a) und (b) alle üblicherweise für derartige Umsetzungen verwendbaren Säurebindemittel eingesetzt werden. Vorzugweise in Frage kommen Alkalihydroxide, wie z.B. Natrium- und Kaliumhydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diaza-bicyclo-[4.3.0]-nonen-5 und 1,8-Diaza-bicyclo-[5.4.0]-undec-7-en (DBU).

Die Reaktionstemperaturen können sowohl bei dem erfindungsgemässen Verfahren (a) als auch bei dem Verfahren (b) jeweils innerhalb eines grösseren Bereiches variiert werden. Im allgemeinen arbeitet man bei den Verfahren (a) und (b) jeweils bei Temperaturen zwischen −20°C und +160°C, vorzugsweise zwischen 0°C und 140°C.

Die erfindungsgemässen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemässen Verfahren (a) und (b) werden die jeweils benötigten Ausgangsstoffe, im allgemeinen in angenähert äquimolaren Mengen eingesetzt. Es ist jedoch auch möglich, eine der beiden jeweils eingesetzten Komponenten in einem grösseren Überschuss zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemässen Verfahren (a) und (b) jeweils nach üblichen Methoden.

Die erfindungsgemässen Wirkstoffe können als Defoliants, Desiccants, Krautabtötungsmittel, und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab. Die erfindungsgemässen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linium, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria,

Eleocharis, Scirpus, Paspalum, Ischaemum, Sphenoclea, Dactyloctenium, Agrostis, Alopecurus, Apera.

Monokotyle Kulturen der Gattungen: Oryza, Zea Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao, Beerenfrucht- und Hopfenanlagen und zur selektiven Unkrautbekämpfung in einjährigen Kulturen eingesetzt werden.

Die erfindungsgemässen Wirkstoffe können in dikotylen Kulturen sowie in Getreide und Reis zur selektiven Unkrautbekämpfung eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Tuluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate und ferner Ammoniumsalze; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azofarbstoffe, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Für die Mischungen kommen bekannte Herbizide wie z.B. 1-Amino-6-ethylthio-3-(2,2-dimethylpropyl)-1,3,5-triazin-2,4 (1H, 3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5 (4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden.

Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemässen Stoffe geht aus den nachfolgenden Beispielen hervor.

## Herstellungsbeispiele

### Beispiel 1

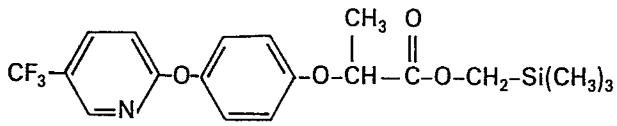

Eine Lösung von 16,5 g (0,05 Mol) 2-[4-(5-Trifluormethylpyridyl-2-oxy)-phenoxy]-propionsäure in 100 ml Aceton wurde bei 20°C unter Rühren mit 8,4 g (0,055 Mol) 1,5-Diaza-bicyclo-[5.4.0] undecen («DBU») versetzt. Das Reaktionsgemisch, in dem sich ein Niederschlag bildete, wurde weitere 30 Minuten bei Raumtemperatur geführt und dann bei 20°C mit 6,1 g (0,05 Mol) Chlormethyltrimethylsilan versetzt. Das Reaktionsgemisch wurde 6,5 Stunden unter Rückfluss gekocht, dann abgekühlt und durch Abziehen des Lösungsmittels eingeengt. Der verbleibende Rückstand wurde in Methylenchlorid gelöst, und die entstehende organische Phase wurde nacheinander mit wässriger Natronlauge, verdünnter wässriger Salzsäure und mit Wasser gewaschen und nach dem Trocknen eingeengt. Man nahm den Rückstand noch einmal in Methylenchlorid auf und reinigte erneut in der zuvor beschriebenen Weise. Man erhielt dadurch 6,4 g (31% der Theorie) an 2-[3-(5-Trifluormethyl-pyridin-2-oxy)-phenoxy]-propionsäure-(trimethylsilyl)-methylester. $n_D^{21,5} = 1,4984$.

Nach der im Beispiel 1 angegebenen Methode erhielt man auch die in den folgenden Beispielen formelmässig aufgeführten Verbindungen.

### Beispiel 2

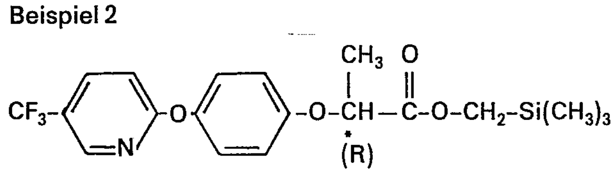

Ausbeute: 63% der Theorie
Brechungsindex: $n_D^{20,5} = 1,4975$
Drehwert: $[\alpha]_D^{24} = + 14,9°$
(1-molare Lösung in Chloroform; Küvettenlänge 10 cm).

### Beispiel 3

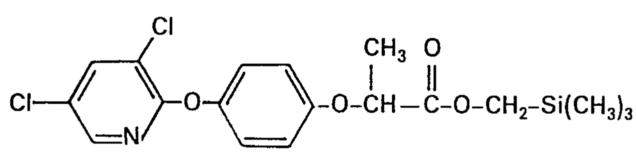

Ausbeute: 87% der Theorie
$n_D^{21,5} = 1,5474$

### Beispiel 4

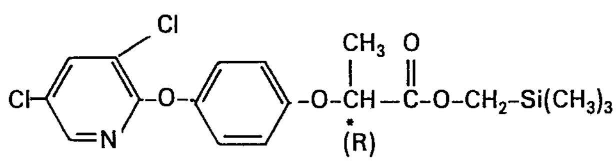

Ausbeute: 24,2% der Theorie
Brechungsindex: $n_D^{23,5} = 1,5433$
Drehwert: $[\alpha]_3^{24} + 15,4°$
(1-molare Lösung in Chloroform; Küvettenlänge 10 cm)

Herstellung von Ausgangsprodukten

Beispiel 5

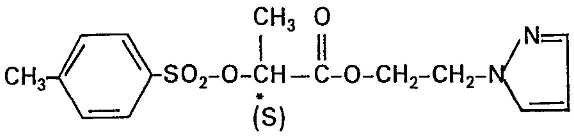

(IV-1)

5,25 g (0,02 Mol) des S-Enantiomeren des Milchsäurechlorid-tosylats wurden bei 20°C unter Rühren in ein Gemisch aus 2,24 g (0,02 Mol) 1-(2-Hydroxyethyl)-pyrazol, 2 g (0,02 Mol) Triethylamin und 50 ml Toluol gegeben. Man rührte weitere 14 Stunden bei 20°C und arbeitete dann auf, indem man das Reaktionsgemisch mit Wasser versetzte, dann mehrfach mit Toluol extrahierte, die vereinigten organischen Phasen trocknete und durch Abziehen des Lösungsmittels unter vermindertem Druck einengte. Man erhielt auf diese Weise 4,7 g (69,5% der Theorie) an dem S-Enantiomeren des 2-Tosyl-oxy-propionsäure-2-(pyrazol-1-yl)-ethyl-esters.

Drehwert: $[\alpha]_D^{24} = -11{,}4°$
(1-molare Lösung in Chloroform; Küvettenlänge 10 cm).

Beispiel 6

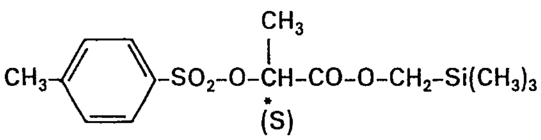

Nach der im Beispiel 5 angegebenen Methode wurde auch die Verbindung der oben aufgeführten Formel hergestellt. Ausbeute: 76% der Theorie

Drehwert: $[\alpha]_D^{24} = -11{,}4°$
(1-molare Lösung in Chloroform; Küvettenlänge 10 cm).

In den nachstehend beschriebenen biologischen Tests wurden die folgenden Verbindungen als Vergleichssubstanzen eingesetzt:

(A) = 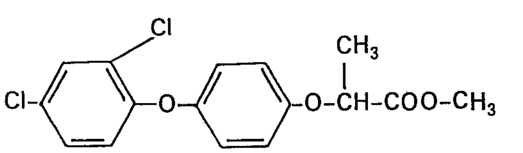

2-[4-(2,4-Dichlorphenoxy)-phenoxy]-propionsäure-methylester (bekannt aus DE-OS 2 223 894).

(B) = 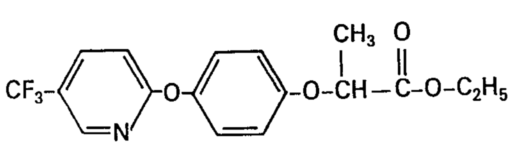

2-[4-(5-Trifluormethyl-pyridyl-2-oxy)-phenoxy]-propionsäure-ethylester (bekannt aus DE-OS 2 812 571).

(C) = 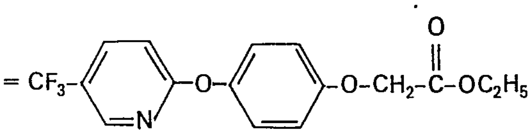

[4-(5-Trifluormethyl-pyridyl-2-oxy)-phenoxy]-essigsäure-ethylester (bekannt aus DE-OS 2 812 571).

Beispiel A
Post-emergence-Test

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigte der erfindungsgemässe Wirkstoff (3) eine bessere selektive herbizide Wirksamkeit als die Vergleichssubstanzen (A), (B) und (C).

Beispiel B
Post-emergence-Test

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, dass die jeweils gewünschten Wirkstoffmengen pro Flächeneinheit ausgebracht werden. Die Konzentration der Spritzbrühe wird so gewählt,

dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigte die Verbindung gemäss Beispiel (4) eine sehr gute selektive herbizide Wirksamkeit.

**Patentansprüche**

1. Substituierte Pyridyl-phenyl-ether der Formel

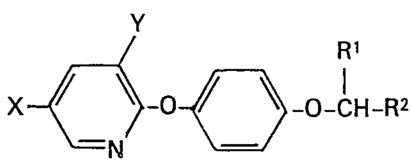

(I)

in welcher
X für Trifluormethyl oder Chlor steht,
Y für Wasserstoff oder Chlor steht,
$R^1$ für Wasserstoff oder Methyl steht, und
$R^2$ für den Rest der Formel

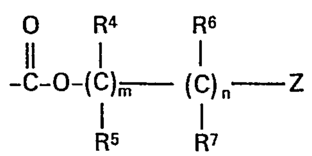

steht, in welcher
$R^4$, $R^5$, $R^6$ und $R^7$ unabhängig voneinander für Wasserstoff oder Alkyl mit 1 bis 4 Kohlenstoffatomen stehen,
m für 1 oder 2 steht,
n für 0 oder 1 steht und
Z für Trimethylsilyl
steht.

2. Substituierte Pyridyl-phenyl-ether der Formel (I) gemäss Anspruch 1, in denen
X für Trifluormethyl oder Chlor steht,
Y für Wasserstoff oder Chlor steht,
$R^1$ für Wasserstoff oder Methyl steht und
$R^2$ für den Rest der Formel

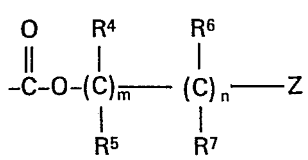

steht, in welcher
$R^4$, $R^5$, $R^6$ und $R^7$ unabhängig voneinander für Wasserstoff oder Methyl stehen,
m für 1 oder 2 steht,
n für 0 oder 1 steht und
Z für Trimethylsilyl steht.

3. R-Enantiomere von substituierten Pyridylphenylethern der Formel (I) gemäss Anspruch 1, in denen
X für Trifluormethyl oder Chlor steht,
Y für Wasserstoff oder Chlor steht,
$R^1$ für Methyl steht und
$R^2$ für den Rest der Formel

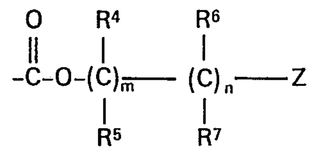

steht, in welcher
$R^4$, $R^5$, $R^6$ und $R^7$ unabhängig voneinander für Wasserstoff oder Methyl stehen,
m für 1 oder 2 steht,
n für 0 oder 1 steht und
Z für Trimethylsilyl steht.

4. Verfahren zur Herstellung von substituierten Pyridyl-phenyl-ethern der Formel

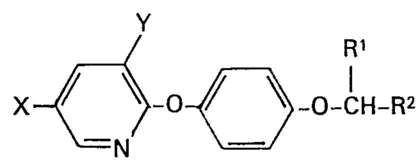

(I)

in welcher
X für Trifluormethyl oder Chlor steht,
Y für Wasserstoff oder Chlor steht,
$R^1$ für Wasserstoff oder Methyl steht und
$R^2$ für den Rest der Formel

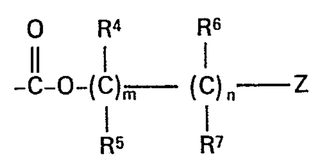

steht, in welcher
$R^4$, $R^5$, $R^6$ und $R^7$ unabhängig voneinander für Wasserstoff oder Alkyl mit 1 bis 4 Kohlenstoffatomen stehen,
m für 1 oder 2 steht,
n für 0 oder 1 steht und
Z für Trimethylsilyl steht, dadurch gekennzeichnet, dass man

a) 4-(Pyridyl-2-oxy)-phenole der Formel

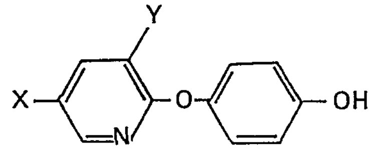

(II)

in welcher
X und Y die oben angegebene Bedeutung haben, mit Alkancarbonsäure-Derivaten der Formel

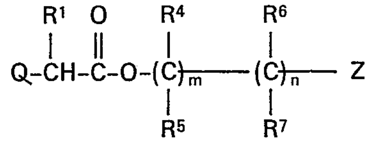

(III)

in welcher
$R^1$, $R^4$, $R^5$, $R^6$, $R^7$, Z, m und n die oben angegebene Bedeutung haben und
Q für Chlor, Brom, Mesylat und Tosylat steht, gegebenenfalls in Gegenwart eines Säurebindemittels und gegebenenfalls in Gegenwart eines Verdünnungsmittels umsetzt, oder

b) Phenoxyalkancarbonsäure-Derivate der Formel

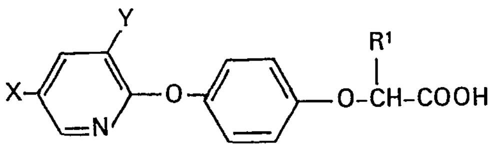 (IV)

in welcher
X, Y und $R^1$ die oben angegebene Bedeutung haben,
mit Silylchloriden

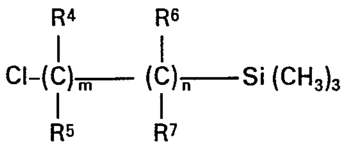 (V)

in welcher
$R^4$, $R^5$, $R^6$, $R^7$, m und n die oben angegebene Bedeutung haben,
gegebenenfalls in Gegenwart eines Säurebindemittels sowie gegebenenfalls in Gegenwart eines Verdünnungsmittels umsetzt.

5. Herbizide Mittel, gekennzeichnet durch einen Gehalt an mindestens einem substituierten Pyridyl-phenylether der Formel (I) gemäss den Ansprüchen 1 bis 4.

6. Verfahren zur Bekämpfung von Unkräutern, dadurch gekennzeichnet, dass man substituierte Pyridyl-phenyl-ether der Formel (I) gemäss den Ansprüchen 1 bis 4 auf die Unkräuter und/oder deren Lebensraum ausbringt.

7. Verwendung von substituierten Pyridyl-phenyl-ethern der Formel (I) gemäss den Ansprüchen 1 bis 4 zur Bekämpfung von Unkräutern.

8. Verfahren zur Herstellung von herbiziden Mitteln, dadurch gekennzeichnet, dass man substituierte Pyridyl-phenyl-ether der Formel (I) gemäss den Ansprüchen 1 bis 4 mit Streckmitteln und/oder oberflächenaktiven Stoffen vermischt.

9. Substituierter Pyridyl-phenyl-ether der Formel

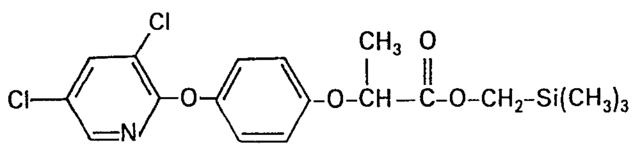

10. R-Enantiomeres des substituierten Pyridyl-phenyl-ethers der Formel

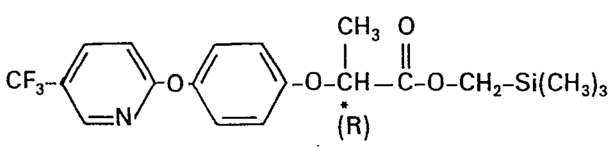

**Claims**

1. Substituted pyridyl-phenyl ethers of the formula

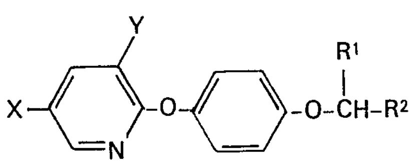 (I)

in which
X represents trifluoromethyl or chlorine,
Y represents hydrogen or chlorine,
$R^1$ represents hydrogen or methyl and
$R^2$ represents the radical of the formula

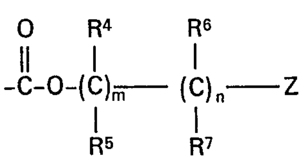

in which
$R^4$, $R^5$, $R^6$ and $R^7$ independently of one another represent hydrogen or alkyl with 1 to 4 carbon atoms,
m represents 1 or 2,
n represents 0 or 1 and
Z represents trimethylsilyl.

2. Substituted pyridyl-phenyl ethers of the formula (I) according to Claim 1, in which
X represents trifluoromethyl or chlorine,
Y represents hydrogen or chlorine,
$R^1$ represents hydrogen or methyl and
$R^2$ represents the radical of the formula

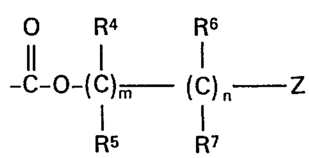

in which
$R^4$, $R^5$, $R^6$ and $R^7$ independently of one another represent hydrogen or methyl,
m represents 1 or 2,
n represents 0 or 1 and
Z represents trimethylsilyl.

3. R-Enantiomers of substituted pyridyl-phenyl ethers of the formula (I) according to Claim 1, in which
X represents trifluoromethyl or chlorine,
Y represents hydrogen or chlorine,
$R^1$ represents methyl and
$R^2$ represents the radical of the formula

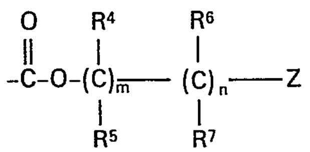

in which
$R^4$, $R^5$, $R^6$ and $R^7$ independently of one another represent hydrogen or methyl,
m represents 1 or 2,
n represents 0 or 1 and
Z represents trimethylsilyl.

4. Process for the preparation of substituted pyridyl-phenyl ethers of the formula

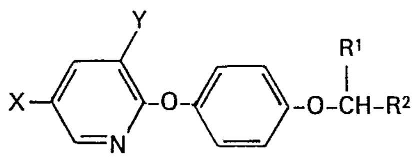 (I)

in which
X represents trifluoromethyl or chlorine,
Y represents hydrogen or chlorine,
$R^1$ represents hydrogen or methyl and
$R^2$ represents the radical of the formula

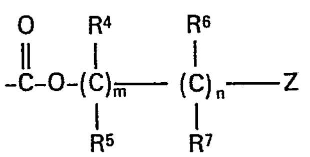

in which
$R^4$, $R^5$, $R^6$ and $R^7$ independently of one another represent hydrogen or alkyl with 1 to 4 carbon atoms,
m represents 1 or 2,
n represents 0 or 1 and
Z represents trimethylsilyl,
characterised in that

a) 4-(pyridyl-2-oxy)-phenols of the formula

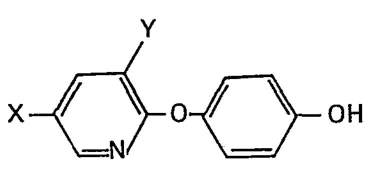 (II)

in which
X and Y have the abovementioned meaning,
are reacted with alkanecarboxylic acid derivatives of the formula

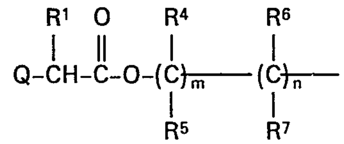 (III)

in which
$R^1$, $R^4$, $R^5$, $R^6$, $R^7$, Z, m and n have the abovementioned meaning and
Q represents chlorine, bromine, mesylate and tosylate,
if appropriate in the presence of an acid-binding agent and
if appropriate in the presence of a diluent,
or

b) phenoxyalkanecarboxylic acid derivatives of the formula

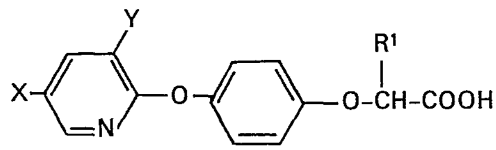 (IV)

in which
X, Y and $R^1$ have the abovementioned meaning, are reacted with silyl chlorides

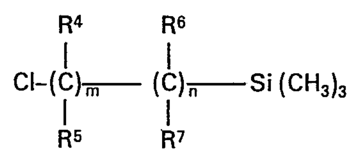 (V)

in which
$R^4$, $R^5$, $R^6$, $R^7$, m and n have the abovementioned meaning,
if appropriate in the presence of an acid-binding agent and if appropriate in the presence of a diluent.

5. Herbicidal agents, characterised in that they contain at least one substituted pyridyl-phenyl ether of the formula (I) according to Claims 1 to 4.

6. Process for combating weeds, characterised in that substituted pyridyl-phenyl ethers of the formula (I) according to Claims 1 to 4 are applied to the weeds and/or their environment.

7. Use of substituted pyridyl-phenyl ethers of the formula (I) according to Claims 1 to 4 for combating weeds.

8. Process for the preparation of herbicidal agents, characterised in that substituted pyridyl-phenyl ethers of the formula (I) according to Claims 1 to 4 are mixed with extenders and/or surface-active substances.

9. Substituted pyridyl-phenyl ethers of the formula

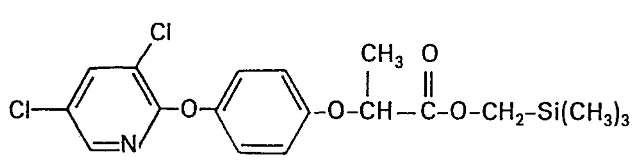

10. R-Enantiomer of the substituted pyridylphenyl ether of the formula

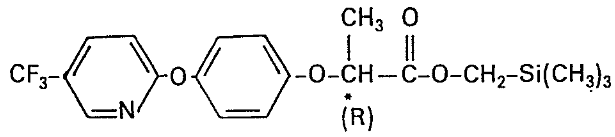

## Revendications

1. Ethers de pyridyle et de phényle substitués de formule

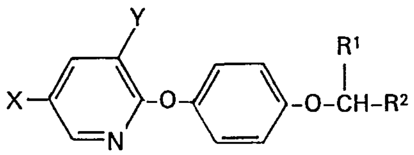

(I)

dans laquelle
X est le groupe trifluorométhyle ou le chlore,
Y est l'hydrogène ou le chlore,
$R^1$ est l'hydrogène ou le groupe méthyle, et
$R^2$ représente les restes de formule

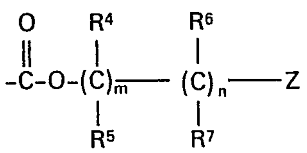

dans laquelle
$R^4$, $R^5$, $R^6$ et $R^7$ représentent indépendamment les uns des autres l'hydrogène ou un groupe alkyle ayant 1 à 4 atomes de carbone,
m est égal à 1 ou à 2,
n est égal à 0 ou à 1 et
Z est le groupe triméthylsilyle.

2. Ethers de pyridyle et de phényle substitués de formule (I) suivant la revendication 1, dans lesquels
X est le groupe trifluorométhyle ou le chlore,
Y est l'hydrogène ou le chlore,
$R^1$ est l'hydrogène ou le groupe méthyle et
$R^2$ est le reste de formule

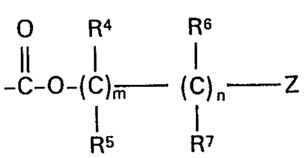

dans laquelle
$R^4$, $R^5$, $R^6$ et $R^7$ représentent indépendamment les uns des autres l'hydrogène ou le groupe méthyle,
m a la valeur 1 ou 2,
n a la valeur 0 ou 1 et
Z est le groupe triméthylsilyle.

3. Enantiomères R d'éthers de pyridyle et de phényle substitués de formule (I) suivant la revendication 1, dans lesquels
X est le groupe trifluorométhyle ou le chlore,
Y est l'hydrogène ou le chlore,
$R^1$ est le groupe méthyle et
$R^2$ est le reste de formule

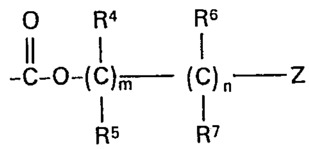

dans laquelle
$R^4$, $R^5$, $R^6$ et $R^7$ représentent indépendamment les uns des autres l'hydrogène ou le groupe méthyle,
m a la valeur 1 ou 2,
n a la valeur 0 ou 1 et
Z est le groupe triméthylsilyle.

4. Procédé de préparation d'éthers de pyridyle et de phényle substitués de formule

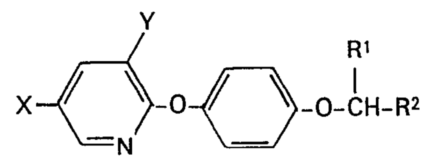

(I)

dans laquelle
X est le groupe trifluorométhyle ou le chlore,
Y est l'hydrogène ou le chlore,
$R^1$ est l'hydrogène ou le groupe méthyle et
$R^2$ est le reste de formule

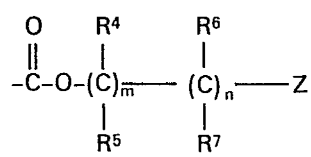

dans laquelle
$R^4$, $R^5$, $R^6$ et $R^7$ représentent indépendamment les uns des autres l'hydrogène ou un groupe alkyle ayant 1 à 4 atomes de carbone,
m a la valeur 1 ou 2,
n a la valeur 0 ou 1 et
Z est le groupe triméthylsilyle, caractérisé en ce que

a) on fait réagir des 4-(pyridyl-2-oxy)-phénols de formule

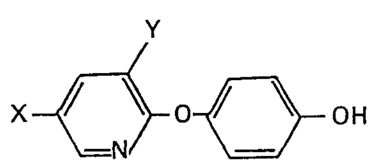

(II)

dans laquelle
X et Y ont la définition indiquée ci-dessus,
avec des dérivés d'acides alcane-carboxyliques de formule

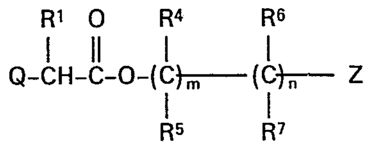

(III)

dans laquelle
$R^1$, $R^4$, $R^5$, $R^6$, $R^7$, Z, m et n ont la définition indiquée ci-dessus, et
Q désigne le chlore, le brome, le groupe mésylate ou tosylate,
éventuellement en présence d'un accepteur d'acide et, le cas échéant, en présence d'un diluant, ou bien

b) on fait réagir des dérivés d'acides phénoxyalcanecarboxyliques de formule

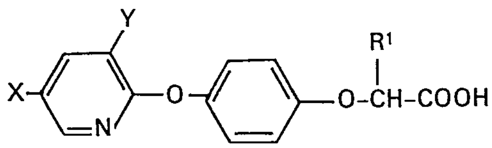

(IV)

dans laquelle,
X, Y et $R^1$ ont la définition indiquée ci-dessus, avec des chlorures de silyle

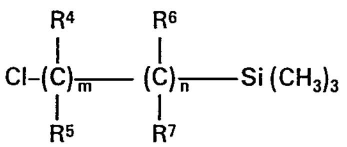

(V)

dans laquelle
$R^4$, $R^5$, $R^6$, $R^7$, m et n ont la définition indiquée ci-dessus,
éventuellement en présence d'un accepteur d'acide ainsi que, le cas échéant, en présence d'un diluant.

5. Compositions herbicides, caractérisées par une teneur en au moins un éther de pyridyle et de phényle substitués de formule (I) suivant les revendications 1 à 4.

6. Procédé pour combattre des mauvaises herbes, caractérisé en ce qu'on applique des éthers de pyridyle et de phényle substitués de formule (I) suivant les revendications 1 à 4 aux mauvaises herbes et/ou à leur milieu.

7. Utilisation d'éthers de pyridyle et de phényle substitués de formule (I) suivant les revendications 1 à 4 pour combattre des mauvaises herbes.

8. Procédé de préparation de compositions herbicides, caractérisé en ce qu'on mélange des éthers de pyridyle et de phényle substitués de formule (I) suivant les revendications 1 à 4 avec des diluants et/ou des agents tensio-actifs.

9. Ethers de pyridyle et de phényle substitués de formule

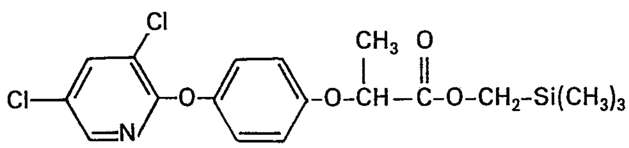

10. L'énantiomère R de l'éther de pyridyle et de phényle substitués de formule